(19) Europäisches Patentamt
European Patent Office
Office européen des brevets

(11) Veröffentlichungsnummer : **0 034 837**
**B1**

(12) # EUROPÄISCHE PATENTSCHRIFT

(45) Veröffentlichungstag der Patentschrift : **15.12.82**

(51) Int. Cl.³ : **C 07 C 21/24**, C 07 C 25/13, C 07 C 25/02// C07C47/542, C07C63/06

(21) Anmeldenummer : **81102900.8**

(22) Anmeldetag : **13.11.79**

(60) Veröffentlichungsnummer der früheren Anmeldung nach Art. 76 EPÜ : **0011281**

(54) **P-tert.-Butylbenzalbromid und dessen am Kern durch Halogen substituierte Derivate.**

(30) Priorität : **16.11.78 DE 2849692**
**22.03.79 DE 2911237**

(43) Veröffentlichungstag der Anmeldung : **02.09.81 (Patentblatt 81/35)**

(45) Bekanntmachung des Hinweises auf die Patenterteilung : **15.12.82 Patentblatt 82/50**

(84) Benannte Vertragsstaaten : **BE CH DE FR GB IT NL**

(56) Entgegenhaltungen : **Keine**

(73) Patentinhaber : **HOECHST AKTIENGESELLSCHAFT**
**Postfach 80 03 20**
**D-6230 Frankfurt/Main 80 (DE)**

(72) Erfinder : **Landauer, Franz, Dr.**
**Liederbacher Strasse 7**
**D-6230 Frankfurt am Main 80 (DE)**
Erfinder : **Schaeffer, Georg, Dr.**
**Herderstrasse 58**
**D-6238 Hofheim am Taunus (DE)**

**EP 0 034 837 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

p-tert.-Butylbenzalbromid und dessen am Kern durch Halogen substituierte Derivate

p-tert.-Butylbenzaldehyd und dessen am Kern durch Halogen substituierte Derivate sind wertvolle Zwischenprodukte für die Herstellung insbesondere von Pharmazeutika, Pflanzenschutzmitteln, Farbstoffen und Riechstoffen.

Die Herstellung des p-tert.-Butylbenzaldehyds und dessen Derivate erfolgt durchweg nach den für die Darstellung aromatischer Aldehyde üblichen Methoden.

So kann man beispielsweise durch Umsetzung von tert.-Butylbenzol mit CO/HCl in Gegenwart von CuCl zu p-tert.-Butylbenzaldehyd gelangen. Der Nachteil dieser Methode besteht jedoch insbesondere in der Notwendigkeit der Verwendung eines Cu-Salzes, wodurch dann eine besondere Abwasserreinigung erforderlich wird.

Ein anderes bekanntes Verfahren, bei dem ebenfalls ein Cu-Salz-$Cu(NO_3)_2$-zum Einsatz kommt, geht aus von p-tert.-Butyltoluol, welches durch Umsetzung einer etwa äquivalenten Menge Brom zunächst in p-tert.-Butylbenzylbromid überführt wird ; daraus wird dann durch längeres Kochen mit einer $Cu(NO_3)_2$-Lösung p-tert.-Butylbenzaldehyd mit einer Ausbeute von etwa 42 % d.Th. gewonnen (J. Chem. Soc., 1935, S. 1848).

Bei einem weiteren Verfahren zur Überführung des p-tert.-Butylbenzylbromids in p-tert.-Butylbenzaldehyd wurde die $Cu(NO_3)_2$-Lösung der letztgenannten Methode durch eine Lösung von Urotropin in wäßrigem Äthanol ersetzt (« Sommelet-Reaktion » ; J. Chem. Soc. 1940, S. 702). Da bei dieser Reaktion aus dem Urotropin ein Gemisch von Methylamin, Ammoniak und Formaldehyd anfällt, dessen Beseitigung aus Umweltschutzgründen notwendig ist, ist auch hier — ebenso wie bei den beiden vorgenannten Cu-Salz-Methoden — eine aufwendige und kostspielige Abwasserreinigung nötig. Außerdem erfordern alle vorerwähnten Methoden einen erheblichen Energieverbrauch und liefern nur unzureichende Raum-Zeit-Ausbeuten.

Die Anwendung einer weiteren für die Herstellung aromatischer Aldehyde bekannten Methode (Verseifung von Benzalchloriden mit Wasser, vergleiche z.B. DE-OS 2 044 832) auf die Herstellung des p-tert.-Butylbenzaldehyds erwies sich, wie eigene Versuche zeigten, als wenig vorteilhaft, weil bei der Herstellung des hier erforderlichen p-tert.-Butylbenzalchlorids durch radikalische Chlorierung von p-tert.-Butyltoluol Produkte erhalten werden, deren organisch gebundenes Chlor nur zum Teil unter den üblichen Bedingungen durch alkalische Hydrolyse wieder abgespalten werden kann (vgl. Houben-Weyl « Methoden der Org. Chemie » Bd. II, S. 233, Stuttgart 1953), was auf eine nicht unbeträchtliche Kernchlorierung und/oder Chlorierung der tert.-Butylgruppe hindeutet. Dies ist — wenn man am Ende reinen p-tert.-Butylbenzaldehyd haben will — natürlich ziemlich unerwünscht. In manchen Fällen werden zwar für die Herstellung von Pharmazeutika, Pflanzenschutzmitteln, Farbstoffen, Riechstoffen, etc. auch am Kern durch Chlor substituierte Derivate des p-tert.-Butylbenzaldehyds benötigt ; das Chlor sollte jedoch gezielt, wie auch die anderen Halogene (F, Br, J), vor der Seitenkettenhalogenierung eingeführt werden.

Es bestand daher die Aufgabe, ein Verfahren zur Herstellung von p-tert.-Butylbenzaldehyd sowie von dessen am Kern durch Halogen substituierten Derivaten zu finden, welches die Nachteile der bekannten Methoden nicht aufweist, welches also keine kostspielige Abwasserreinigung erfordert, gute Ausbeuten an dem gewünschten Produkt und keine in unerwünschter Weise substituierten Nebenprodukte liefert und auch sonst in jeder Beziehung wirtschaftlich ist.

Diese Aufgabe konnte erfindungsgemäß in einfacher Weise über die Schaffung des neuen Zwischenproduktes p-tert.-Butylbenzalbromid und dessen am Kern durch Halogen (mono-) substituierte Derivate gelöst werden. Die neuen Produkte besitzen die Formel I

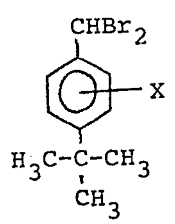

(I)

worin X = H, F, Cl, Br, J, vorzugsweise H, F, Cl, Br, insbesondere H.

Sie werden dadurch hergestellt, daß man p-tert.-Butyltoluol und dessen am Kern durch Halogen substituierte Derivate mit etwa 2 Mol Brom/Mol organisches Ausgangsmaterial bei Temperaturen von etwa 40 bis 200, vorzugsweise etwa 40 bis 120 °C, ggf. unter der Einwirkung energiereicher Strahlung oder in Gegenwart von Radikalbildnern umsetzt.

Die Weiterverarbeitung des dabei gebildeten p-tert.-Butylbenzalbromids und dessen am Kern durch Halogen substituierter Derivate zu p-tert.-Butylbenzaldehyd und dessen am Kern durch Halogen substituierten Derivaten erfolgt durch Verseifung mit Wasser, gegebenenfalls in Gegenwart von üblichen Verseifungskatalysatoren.

Als Ausgangsmaterialien für die Herstellung der neuen Verbindungen der Formel I dienen p-tert.-Butyltoluol und dessen am Kern durch Halogen (mono-) substituierte Derivate der folgenden allgemeinen Formel II

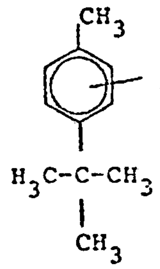

(II)

worin X die gleiche Bedeutung wie in Formel I besitzt.

Das jeweilige Ausgangsprodukt wird mit etwa 2 (1,8 bis 2,2, insbesondere 1,9-2,1) Mol Brom/Mol Ausgangsmaterial bei den angegebenen Temperaturen — etwa 40 bis 200 °C, vorzugsweise etwa 40 bis 120 °C — gegebenenfalls unter Einwirkung energiereicher Strahlung oder in Gegenwart von Radikalbildnern umgesetzt. Als energiereiche Strahlung kommt vorzugsweise UV-Licht infrage.

Geeignete Radikalbildner sind die für Seitenkettenchlorierungen üblichen organischen Peroxide, Azoisobutyronitril, etc.

Die energiereiche Strahlung oder die Gegenwart von Radikalbildern sind für das Gelingen der Reaktion zwar nicht unbedingt erforderlich, jedoch in erheblichem Maß reaktionsbeschleunigend und daher vorteilhaft.

Das Brom kann bei dieser Reaktion entweder flüssig zugetropft oder gasförmig (nach dem Verdampfen) eingeleitet werden, wobei im letzteren Fall auch ein Inertgas (Stickstoff, Argon, etc.) zugesetzt werden kann.

Die Bromierung Kann sowohl ohne als auch in einem geeigneten Lösungsmittel durchgeführt werden, wobei als Lösungsmittel inerte — insbesondere halogenierte — Kohlenwasserstoffe wie z.B. $CCl_4$ oder o-Dichlorbenzol in Betracht kommen.

Weiterhin ist die Durchführung der Reaktion drucklos oder unter erhöhtem Druck sowie sowohl diskontinuierlich als auch kontinuierlich möglich.

Das bei dieser Bromierung gebildete p-tert.-Butylbenzalbromid sowie — wenn die am Kern durch Halogen substituierten Derivate des p-tert.-Butyltoluols als Ausgangsstoffe verwendet wurden — die entsprechenden am Kern durch Halogen substituierten Derivate, das sind also die erfindungsgemäßen Verbindungen der Formel I, besitzen keine unerwünschten Bromsubstituenten am aromatischen Kern und in der ter.-Butylgruppe.

Die unter Formel I fallenden Verbindung sind :

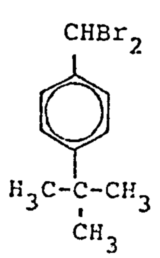
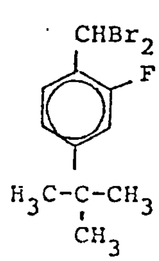
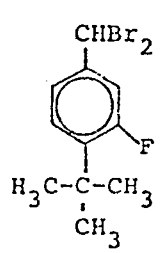
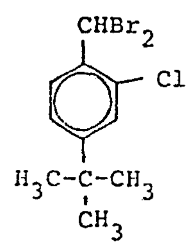
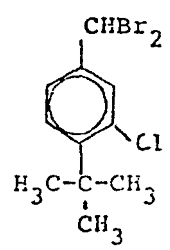
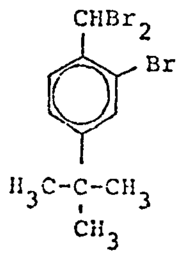
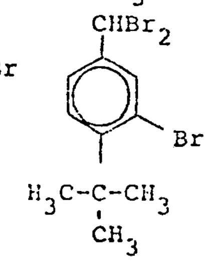
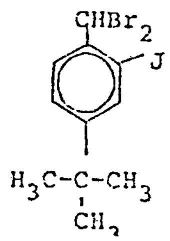

und

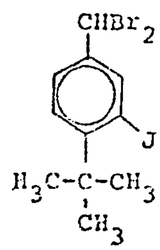

Für die Weiterverarbeitung zum entspr. Benzaldehyd können die Verbindungen (I) entweder isoliert und gereinigt (z.B. durch Umkristallisation) oder ohne Isolierung verseift werden.

Die Verseifung erfolgt nach den für die Verseifung von Benzalhalogeniden üblichen Methoden bei erhöhter Temperatur — vorzugsweise bei Temperaturen von etwa 60 bis 150, insbesondere etwa 80 bis 120 °C — mit Wasser, gegebenenfalls in Gegenwart von üblichen Verseifungskatalysatoren, z.B. von Metallhalogeniden wie $FeCl_3$, $ZnBr_2$, etc., oder auch von $H_2SO_4$ und dgl. Vorzugsweise wird pro Mol

Benzalbromid (I) ein Mol Wasser verwendet; es kann aber auch ein Überschuß an Wasser eingesetzt werden, wobei jedoch dann eine zusätzliche Abtrennung der wäßrigen Phase durchzuführen ist. In der Regel wird zu vorgelegtem Benzalbromid (+ Verseifungskatalysator) Wasser in dem Maß zugegeben, in welchem sich Bromwasserstoff bildet und anschließend absorbiert werden kann.

Die Verseifung kann im übrigen z.B. sowohl drucklos als auch unter erhöhtem Druck durchgeführt werden. Man kann auch inerte Lösungsmittel wie z.B. Kohlenwasserstoffe oder Chlorkohlenwasserstoffe zusetzen. Ferner ist noch etwa die Verwendung von Emulgatoren möglich, die eine gute Vermischung der organischen Phase mit dem zugegebenen Wasser bewirken.

Die Absorption des Bromwasserstoffs erfolgt zweckmäßig in einer üblichen Absorptionsanlage; er kann dann direkt für weitere chemische Reaktionen verwendet oder verkauft werden.

Auf diese Weise werden p-tert.-Butylbenzaldehyd und dessen am Kern durch Halogen substituierte Derivate — die Produkte besitzen die allgemeine Formel III

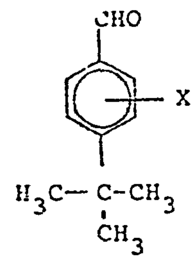

(III)

worin X = H, F, Cl, Br, J, vorzugsweise = H, F, Cl, Br, insbesondere = H.
in ausgezeichneten Ausbeuten erhalten. Die Produkte besitzen — außer den schon in den Ausgangsmaterialien vorhandenen Kernsubstituenten — keine unerwünschten (Brom-) Substituenten mehr. Diese Tatsache ist außerordentlich überraschend, da Halogenierungen mit Chlor und Brom normalerweise immer gleichartig verlaufen, in diesem Fall, bei Verwendung von Chlor, aber durch Seitenkettenchlorierung von p-tert.-Butyltoluol eine beträchtliche Bildung von Nebenprodukten nicht zu vermeiden ist. Im Gegensatz dazu findet bei der Bromierung des p-tert.-Butyltoluols (und dessen entsprechender Substitutionsprodukte) keine oder so gut wie keine unerwünschte Kernhalogenierung und darüberhinaus auch keine Bromierung der tert.-Butylgruppe statt. Die hohe Selektivität und Ausbeute des Verfahrens sowie die Tatsache, daß — im Gegensatz zu etlichen Verfahren des Standes der Technik — keinerlei besondere Abwasseraufbereitung erforderlich ist, stellen einen erheblichen Fortschritt dar.

Die Erfindung wird nun anhand der folgenden Beispiele näher erläutert. Auf die (Erfindungs-) Beispiele folgt dann ein Vergleichsbeispiel, bei welchem als Halogenierungsmittel für das p-tert.-Butyltoluol anstelle des Broms Chlor verwendet wurde.

## Beispiel 1

In einem 1 l Vierhalskolben mit Rührer, Thermometer, Tropftrichter und Rückflußkühler, der mit einer (mit Wasser gefüllten) Absorptionsvorrichtung für Bromwasserstoff verbunden ist, werden 296 g p-tert.-Butyltoluol (2 Mol) auf 100 bis 110 °C erhitzt. Unter Bestrahlung mit UV-Licht tropft man dann im Verlauf von 3,5 Stunden 656 g Brom (4,1 Mol) zu. Anschließend wird mit Stickstoff der noch im Reaktionsprodukt befindliche Bromwasserstoff ausgeblasen.

Rohausbeute p-tert.-Butylbenzalbromid : 606 g (= 98,7 % d.Th.).
gef. : Gasamt-Brom : 53,3 %
verseifbares Brom : 53,4 % ; ber. : 52,3 %.

Eine gleich gute Ausbeute wird erhalten, wenn das Brom gasförmig eingeleitet wird.

Fp. des reinen p-tert.-Butylbenzalbromids : 44 °C (umkristallisiert aus Äthanol).

Weiterverarbeitung (Verseifung) zum p-tert.-Butylbenzaldehyd :

Zu dem rohen p-tert.-Butylbenzalbromid gibt man 0,6 g $ZnCl_2$ und 0,3 g Wasser, erhitzt auf 110 °C und läßt im Verlauf von 4 Stunden 36 g Wasser zutropfen, wobei die Reaktionstemperatur allmählich auf 90 bis 100 °C gesenkt wird. Der bei der Verseifung entstehende Bromwasserstoff wird in einer nachgeschalteten Apparatur in Wasser aufgefangen.

Nach Zugabe des Wassers wird 30 Minuten nachgerührt und der restliche Bromwasserstoff mit Stickstoff ausgeblasen. Zurückerhaltener Bromwasserstoff : 316 g (= 97,5 % d.Th.).

Der rohe p-tert.-Butylbenzaldehyd wird anschließend im Vakuum ($Kp_3$ : 90 °C) destilliert.

Ausbeute : 299 g = 92,5 % d.Th. (= 1,85 Mol).

## Beispiel 2

In einem 250 ml Glaskolben wurden 100 g = 0,548 Mol 1-Methyl-2-Chlor-4-tert-butyl-benzoyl unter UV-Bestrahlung und unter Rühren mit 175,5 g = 1,097 Mol Brom folgendermaßen zur Reaktion gebracht :

Bei einer Reaktionstemperatur von 130 °C wurden innerhalb 60 Minuten 100 g = 0,625 Mol flüssiges

Brom eingetropft. Dann wurde die Reaktionstemperatur auf 180 °C erhöht und innerhalb 30 Minuten wurden weitere 75,5 g = 0,472 Mol Brom eingetropft. Bei 180 °C wurde dann noch 30 Minuten lang nachgerührt. Dann wurde das Reaktionsgemisch auf 20 °C abgekühlt und von darin gelösten geringen Mengen Bromwasserstoff und Brom mittels Durchleiten eines Stickstoffstromes befreit.

Nach dem Ausblasen wurden 186 g einer rohen Lösung erhalten, die zu 85 % aus 2-Chlor-4-tert-butylbenzalbromid bestand. Die Identität des Produktes wurde durch Massenspektren und NMR-Spektren bestätigt, die an dem destillierten Produkt ($Kp._{0,2}$ 115 °C) aufgenommen wurden.

Vergleichsbeispiel

In genau der gleichen Apparatur und unter den gleichen Bedingungen wie in (Erfindungs-) Beispiel 1 wurde, 296 g p-tert.-Butyltoluol (2 Mol) durch Einleiten von 284 g (ca. 4,1 Mol) Chlor im Verlauf von 3,5 Stunden chloriert. Nach dem Ausblasen des Chlorwasserstoffs betrug die Rohausbeute 415 g ; darin wurden gefunden :

Gesamtchlor : 31,05 %

verseifbares Chlor : 20,85 % (Bestimmung : Verseifung mit alkoholischer KOH und argentometrische Bestimmung des ionogenen Chlors) ;

daraus berechnet sich an nicht abspaltbarem Chlor : 10,20 %.

Etwa 1/3 des gesamten organisch gebundenen Chlors ist also für die Verseifung zum Aldehyd wertlos.

Ein sehr ähnliches Ergebnis wurde erhalten, als zur Chlorierung anstelle des Chlors die äquivalente Menge Sulfurylchlorid verwendet wurde.

**Anspruch**

p-tert.-Butylbenzalbromid und dessen am Kern durch Halogen substituierte Derivate der Formel I

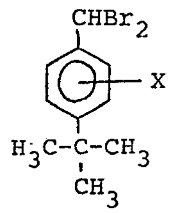

(I)

worin X = H, F, Cl, Br, J, vorzugsweise H, F, Cl, Br, insbesondere = H.

**Claim**

p-tert.-butylbenzalbromide and its derivatives substituted by halogen at the nucleus of the formula I

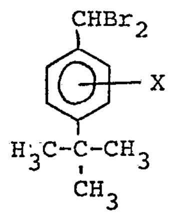

(I)

wherein X = H, F, Cl, Br, J, preferably H, F, Cl, Br, especially = H.

**Revendication**

Bromure de p-tert.-butyl-benzylidène et ses dérivés substitués par un halogène sur le noyau, qui répondent à la formule I

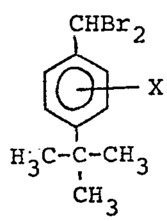

(I)

dans laquelle X représente H, F, Cl, Br, ou I, de préférence H, F, Cl ou Br, plus spécialement H.